# EUROPÄISCHE PATENTSCHRIFT

(11) **EP 0 378 592 B1**
(45) Veröffentlichungstag und Bekanntmachung des Hinweises auf die Patenterteilung: **01.03.1995**
(21) Anmeldenummer: 89904010.9
(22) Anmeldetag: 13.04.1989
(51) Int. Cl.: C12P 13/04, C12P 13/02

(54) **VERFAHREN ZUR HERSTELLUNG VON L-AMINOSÄUREN UND AMINOSÄURE-AMIDEN**
PROCESS FOR PRODUCING L-AMINO ACIDS AND AMINO ACID AMIDES
PROCEDE DE FABRICATION DE L-AMINOACIDES ET D'AMIDES D'AMINOACIDE

(30) Priorität: 06.05.1988 DE 3816063
(43) Veröffentlichungstag der Anmeldung: 25.07.1990
(73) Patentinhaber: SCHERING AKTIENGESELLSCHAFT, 13342 Berlin (DE)
(72) Erfinder: KLAGES, Uwe, D-1000 Berlin 28 (DE); WEBER, Alfred, D-1000 Berlin 37 (DE)
(86) Internationale Anmeldenummer: DE8900232
(87) Internationale Veröffentlichungsnummer: WO8910969

(56) Entgegenhaltungen:
- EP-A- 193 113
- EP-A- 199 407
- WO-A-80/01571
- WO-A-86/07386

## Beschreibung

Die Erfindung betrifft das in den Patentansprüchen gekennzeichnete Verfahren zur Herstellung von L-Aminosauren und Aminosäure-amiden.

Verfahren zur fermentativen Herstellung von L-Aminosäuren aus D,L-α-Aminonitrilen und D,L-Aminosäureamiden sind vorbekannt (EP-A 23214, WO 86/07386 und WO 80/01571). Bei diesen vorbekannten Verfahren wird die L-Form des α-Aminonitrils beziehungsweise α-Aminosäureamids zur L-Aminosäure hydrolysiert, während die D-Form des α-Aminonitrils oder α-Aminosäureamids nicht hydrolysiert oder nur zum D-Aminosäureamid hydrolysiert wird. Dies bedingt, daß mittels der vorbekannten Verfahren theoretisch nur Ausbeuten von 50% erzielt werden können. Bekannt sind ferner Verfahren, die unerwünschten optisch aktiven Aminosäureamide zu racemisieren, um die so erhaltenen Racemate erneut in die optischen Antipoden aufzuspalten (EP-A 0193113 und EP-A 0199407).

Es wurde nun gefunden, daß gewisse Mikroorganismen nicht nur L-AminosäureamidAmidasen besitzen, sondern darüber hinaus auch Aminosäuramid-Racemasen haben, die die D-Aminosäure-amide zu D.L-Aminosäureamiden razemisieren. Diese Mikroorganismen haben somit die Fähigkeit auch die D-Form der D,L-Aminosäure-amide in L-Aminosäuren umzuwandeln. Die Auffindung derartiger Mikroorganismen bereitet dem Fachmann keine Schwierigkeiten. Mittels der üblichen Screening Methoden werden unterschiedliche Mikroorganismen getestet, ob diese die Fähigkeit besitzen D-Aminosäureamide in L-Aminosäuren umzuwandeln. Vorbekannte Mikroorganismen, die diese Fähigkeit in hervorragendem Maße besitzen sind nach eigenen Untersuchungen Arthrobacter sp. ATCC 31 652 und Corynebacterium sp. ATCC 31 662.

Es wurde ferner gefunden, daß es Mikroorganismen gibt, die die Fähigkeit besitzen, α-Aminonitrile in Aminosäure-amide zu überführen, ohne diese weiter zu hydrolysieren. Aus Bodenproben wurde ein Mikroorganismus isoliert, der, die Fähigkeit besitzt, D,L-α-Amino-β-phenylpropionitril zu Phenylalanin zu spalten.

Dieser Mikroorganismus hat folgende taxonomische Eigenschaften:

| | |
|---|---|
| Koloniemorphologie | rund, glattrandig durchscheinend, schleimig |
| Zellmorphologie | unbewegliche Kurzstäbchen 1,5-2,5 »m lang, 1»m breit |
| Gram-Färbung | gram-negativ |
| Endosporen | negativ |
| Katalase | positiv |
| Cytochrom-C-Oxidasen | negativ |
| Citrat-Verwertung | positiv |
| Nitrit aus Nitrat | negativ |
| Voges-Proskauer | negativ |
| Methylrot | negativ |
| Indol-Bildung | negativ |
| Urease | positiv |
| H₂S-Bildung | negativ |
| Zucker-Verwertung | Säure aus Glukose, keine Säure aus |
| | Saccharose, Fructose, Lactose, Maltose, |
| | Mannit, Glycerin, keine Gasbildung |

Aufgrund seiner morphologischen und physiologischen Eigenschaften wurde der Mikroorganismus nach den Kriterien von "Bergey's Manual of Determinative Bacteriology" als Acinetobacter calcoaceticus bestimmt.

Dieser Mikroorganismus wurde mittels N-Methyl-N'-nitro-N-nitrosoguanidin (MNNG) mutiert und eine amidasefreie Mutante isoliert, die die Fähigkeit besitzt, D,L-α-Aminonitrile nur noch bis zur Stufe der D,L-Aminosäureamiden zu hydrolysieren. Diese Mutante wurde bei der "Deutschen Sammlung von Mikroorganismen" unter der Nummer DSM 3875 deponiert.

Mit Hilfe des neuen Mikroorganismus Acinetobacter calcoaceticus DSM 3875 sowie der vorbekannten Mikroorganismen Arthrobacter sp. ATCC 31652 oder Corynebacterium sp. ATCC 31 662 ist es nun möglich, D,L-α-Aminonitrile zu L-Aminosäuren zu hydrolysieren, ohne daß als Nebenprodukte D-Aminosäureamide zurückbleiben.

Aminosäuren die mittels des erfindungsgemäßen Verfahrens synthetisiert werden können sind beispielsweise die in Hoppe-Seyler's Z.Physiol. Chem. 348, 1967, 256-261 aufgeführten Aminosäuren sowie deren Derivate, bei denen gegebenenfalls im Rest A gemäß Formel I vorhandene Hydroxygruppen, Thiolgruppen, Aminogruppen und Carboxylgruppen durch geeignete Schutzgruppen blockiert sind.

Als geeignete Alkylreste A seien beispielsweise die Methylgruppe, die Ethylgruppe, die Propylgruppe, die 1-Methylethylgruppe, die Butylgruppe, die 1-Methylpropylgruppe, die 2-Methylpropylgruppe, die 1,1-Dimethylethylgruppe, die Pentylgruppe, die 1-Methylbutylgruppe, die 3-Methylbutylgruppe oder die Hexylgruppe tragen. Besonders bevorzugte Alkylgruppen A sind solche mit maximal 6 Kohlenstoffatomen, wie die Methylgruppe des Verfahrensprodukts Alanin, die 1-Methylethylgruppe des Valins, die 2-Methylpropylgruppe des Leucins, die 1-Methylpropylgruppe des Isoleucins oder die Ethylgruppe der α-Aminobuttersäure.

Die Alkylgruppen A können gegebenenfalls durch Hydroxygruppen, Mercaptogruppen, Aminogruppen oder Carbonylgruppen substituiert sein, wobei einfach substituierte Alkylgruppen bevorzugt sind, oder durch Sauerstoffatome (vorzugsweise eins), Stickstoffatome (vorzugsweise eins oder zwei) oder Schwefelatome (vorzugsweise eins) unterbrochen sein. Als Alkylgruppen, die durch Hydroxygruppen oder Mercaptogruppen substituiert sind, seien besonders hervorgehoben die Hydroxymethylgruppe des Serins, die 1-Hydroxy ethylgruppe des Thereonins, die Mercaptomethylgruppe des Cysteins, die 2-Mercaptoethylgruppe des Homocysteins und die 1-Mercapto-1-methyl-ethylgruppe des β-Thiovalins. Als eine durch ein Schwefelatom unterbrochene Alkylgruppe A sei die 2-Methylthioethylgruppe des Methionins hervorgehoben. Alkylgruppen A, die als Substituenten eine Aminogruppe tragen sind beispielsweise die 2-Aminoethylgruppe der α,γ-Diaminobuttersäure, die 3-Aminopropylgruppe des Ornithins und die 4-Aminobutylgruppe des Lysins. Durch Carboxylgruppen substituierte Alkylgruppen A ist beispielsweise die 2-Carboxyethylgruppe der Glutaminsäure.

Als durch Hydroxygruppen substituierte Phenylreste, Benzylreste oder 3-Indolylmethylreste A seien beispielsweise genannt, der 4-Hydroxyphenylrest, der 4-Hydroxyphenylmethylrest und der 5-Hydroxy-3-indolylmethylrest.

Die Erfindung betrifft sowohl das in dem Patentanspruch 1 gekennzeichnete Verfahren zur Herstellung von L-Aminosäuren der allgemeinen Formel I aus den α-Amino-nitrilen der allgemeinen Formel II, als auch das in dem Patentanspruch 2 gekennzeichnete Verfahren zur Herstellung von Aminosäureamiden der allgemeinen Formel III und das in dem Patentanspruch 3 gekennzeichnete Verfahren zur Herstellung von L-Aminosäuren aus diesen Amiden.

Zur Durchführung dieser Verfahren wird der verwendete Mikroorganismus unter den üblicherweise verwendeten Kulturbedingungen in einem geeigneten Nährmedium unter Belüften, Submerskulturen angezüchtet. Dann setzt man der Kultur das Substrat (vorzugsweise in einem geeigneten Lösungsmittel gelöst) zu und fermentiert, bis eine maximale Substratumwandlung erreicht ist.

Geeignete Substratlösungsmittel sind beispielsweise Wasser, Methanol, Ethanol, Glykolmonomethylether, Dimethylformamid oder Dimethylsulfoxyd.

Die optimale Substratkonzentration, Substratzugabezeit und Fermentationsdauer ist von der Struktur des verwendeten Substrates und der Art der verwendeten Fermentationsbedingungen abhängig. Diese Größen müssen, wie dies bei mikrobiologischen Fermentationen allgemein erforderlich ist, im Einzelfall durch Vorversuche, wie sie dem Fachmann geläufig sind, ermittelt werden. Während der Fermentation wird der pH-Wert der Fermentationsbrühe vorzugsweise auf einen pH-Wert von 7,5-10 eingestellt.

Andererseits ist es aber auch möglich und wegen der starken Polarität der Verfahrensprodukte oft vorteilhaft, den angezüchteten Mirkoorganismus beispielsweise durch Filtration oder Zentrifugieren vom Kulturmedium abzutrennen und die Fermentation der Substrate mit der isolierten Zellmasse im resting cell Verfahren durchzuführen.

Ferner ist zu erwarten, daß das erfindungsgemäße Verfahren auch sehr gut mittels immobilisierter Mikroorganismen durchgeführt werden kann.

Die Immobilisierung der Mikroorganismen durch Einbetten in ein die Mikroorganismen nicht denaturierendes Polymeres erfolgt nach Methoden, die dem Fachmann wohl bekannt sind. (I. Chibata Immobilized Enzymes; Reserch and Development; 1978; S.P. Colowick und N.O. Kaplan, Methods in Enzymology, Academic Press, New York, et al. Vol 44, 1976 und Bo Matthiasson Immobilised Cells and Organells, CRC Press Inc., Boca Raton, Florida Vol. 1 und 2.)

### Geeignete Immobilisate sind wahrscheinlich:

### Mikrooganismen-Immobilisate auf Alginat-Basis.

Diese lassen sich herstellen, indem man die Mikkroorganismen in einer wässrigen Vorpolymeren-Lösung enthaltend 0.5 bis 5 Gewichtsprozent Natriumalginat suspendiert und diese Suspension bei einer Temperatur von 5 bis 40° C unter Rühren in eine 0,01 bis 0,4 molare wässrige Lösung eines Aluminium oder vorzugsweise Calziumsalzes (beispielsweise Calziumchlorid) einträgt.

### Mikroorganismen-Immobilisate auf Carragenan-Basis.

Diese lassen sich beispielsweise herstellen, indem man die Mikroorganismen in einer auf 25 bis 50° C erwärmte 0,1 bis 1,0 gewichtsprozentige wässrige Carragenan-Lösung suspendiert, die Lösung erkalten läßt bis sie gelartig wird, das Gel mechanisch zerkleinert und dann in einer 0,2 bis 2,0 gewichtsprozentige wässrigen Lösung eines Kaliumsalzes (beispielsweise Kaliumchlorid) härtet.

### Mikroorganismen-Immobilisate auf Chitosan-Basis.

Diese können beispielsweise hergestellt werden, indem man die Mikroorganismen in einer auf 20° C bis 40° C erwärmten 0.5 bis 13 %igen protonierten (eingestellt auf pH 4,5 bis 5,5) Chitosan-Lösung suspendiert und in diese eine Gegenionenlösung von beispielsweise Kaliumhexacyanoferat(II) (mit einer Konzentration von 0,01 bis 1 mol/Liter) eintropft.

Das erfindungsgemäße Verfahren gemäß Patentanspruch 1 kann in der Weise durchgeführt werden, daß man die Mikroorganismen nacheinander oder gleichzeitig auf die α-Amino-nitrile der Formel II einwirken läßt. Läßt man die unterschiedlichen Mikroorganismen gleichzeitig auf die Substrate einwirken, so ist es zweckmäßig die Zellen von Acinetobacter calcoanticus und Arthrobacter sp. bzw. Corynebacterium sp. in einem Verhältnis von 1:2 bis 1:20 miteinander zu mischen.

### Beispiel 1

a) Ein 500 ml Erlenmeyerkolben mit 100 ml sterilem Nährmedium, enthaltend
   0,05 g Trinatriumcitrat
   0,35 g Dikaliumhydrogenphosphat
   0,01 g Magnesiumsulfat-Heptahydrat
   0,1 g Popionsäureamid
   wird mit einer Kultur von Acinetobacter calcoaceticus DSM 3875 beimpft und 20 Stunden lang bei 30° C mit 180 Umdrehungen pro Minute geschüttelt.
   Dann zentrifugiert man die Zellen ab und resuspendiert sie in 20 ml eines 0,1 molaren Kaliumphosphat-Puffer von pH 7,0.
b) Ein 500 ml Erlenmeyerkolben mit 100 ml steriler Nährlösung enthaltend
   0,05 g Trinatriumcitrat
   0,35 g Dikaliumhydrogenphosphat
   0,15 g Kaliumdihydrogenphosphat
   0,01 g Magnesiumsulfat-Heptahydrat
   0,1 g Ammoniumsulfat
   wird mit Arthrobacter spec. ATCC 31652 beimpft und 20 Stunden lang bei 30° C mit 180 Umdrehungen pro Minute geschüttelt. Dann werden die Zellen abzentrifugiert und in 20 ml 0,1 molarem Kaliumphosphat-Puffer von pH 7 resuspendiert.
c) 0,5 ml Zellsuspension von Acinetobacter calcoaceticus DSM 3875 - hergestellt nach Beispiel a) - werden mit 1,5 ml Zellsuspension von Arthrobacter spec. ATCC 31652 - hergestellt nach Beispiel b) - gemischt und mit 2 mg D,L-α-Amino-β-phenylpropionitril versetzt und 4 Stunden lang bei 30° C gerührt. Die erhaltene Reaktionsmischung wird dünnschichtchromatographisch und durch Umsetzung mit L-und D-Aminosäureoxydase auf ihren Gehalt an Aminosäuren bestimmt. Sie enthält 1,2 mg L-Phenylalanin aber kein D-Phenylalanin.

### Beispiel 2

Unter den Bedingungen des Beispiels 1, aber unter Verwendung von Corynebacterium sp. ATTC 31662 anstelle von Arthrobacter sp. ATCC 31652 werden
2 mg D,L-α-Amino-β-phenylpropionitril umgesetzt und es sind analytisch 1.3 mg L-Phenyalanin aber kein D-Phenylalanin in der Reaktionsmischung nachweisbar.

### Beispiel 3

a) Unter den Bedingungen des Beispiels 1b werden 100 ml einer Kultur von Arthrobacter sp. ATCC 31652 angezüchtet. Die erhaltenen Zellen werden abzentrifugiert und in 100 ml 0,1 molaren Kaliumphosphat-Puffer resuspendiert.
b) 5 ml der Bakterien-Suspension werden mit 5 mg D-Phenylalaninamid versetzt und 4 Stunden lang bei 30° C gerührt. In der Reaktionsmischung sind analytisch 2,1 mg L-Phenylalanin aber kein D-Phenylalanin nachweisbar.

### Beispiel 4

a) Unter den Bedingungen des Beispiels 1b werden 100 ml einer Kultur von Corynebacterium sp. ATCC 31662 angezüchtet. Die erhaltenen Zellen werden abzentrifugiert und in 100 ml 0.1 molaren Kaliumphosphat-Puffer resuspendiert.
b) 5 ml der Bakterien-Suspension werden mit 5 mg D-Phenylalaninamid versetzt und 4 Stunden lang bei 30° C gerührt. In der Reaktionsmischung sind 1.8 mg L-Phenylalanin aber kein D-Phenylalanin nachweisbar.

### Beispiel 5

Unter den Bedingungen des Beispiels 3 werden 5 mg DL-Tryptophanamid umgesetzt und es bilden sich 1,5 mg L-Tryptophan aber kein D-Tryptophan.

### Beispiel 6

Unter den Bedingungen des Beispiels 4 werden 5 mg D,L-Tryptophanamid umgesetzt und es bildet sich 1,3 mg L-Tryptophan aber kein D-Tryptophan.

### Beispiel 7

Unter den Bedingungen des Beispiels 3 werden 5 mg D,L-Leucinamid umgesetzt und es bilden sich 2,2 mg L-Leucin aber kein D-Leucin.

### Beispiel 8

Unter den Bedingungen des Beispiels 4 werden 5 mg D,L-Leucinamid umgesetzt und es bilden sich 2,4 mg L-Leucin aber kein D-Leucin.

### Beispiel 9

Unter den Bedingungen des Beispiels 3 wird D,L-Alaninamid umgesetzt und es bildet sich L-Alanin aber kein D-Alanin.

### Beispiel 10

Unter den Bedingungen des Beispiels 4 wird D,L-Alaninamid umgesetzt und es bildet sich L-Alanin aber kein D-Alanin.

### Beispiel 11

Unter den Bedingungen des Beispiels 3 wird D,L-Valinamid umgesetzt und es bildet sich L-Valin aber kein D-Valin.

### Beispiel 12

Unter den Bedingungen des Beispiels 4 wird D,L-Valinamid umgesetzt und es bildet sich L-Valin aber kein D-Valin.

### Beispiel 13

Unter den Bedingungen des Beispiels 3 wird D,L-Methioninamid umgesetzt und es bildet sich L-Methionin aber kein D-Methionin.

Unter den Bedingungen des Beispiels 4 wird D,L-Methioninamid umgesetzt und es bildet sich L-Methionin aber kein D-Methionin.

### Beispiel 15

5 mg D,L-α-Amino-β-phenyl-propionitril werden mit 5 ml Zellsuspension von Acinetobacter calcoaceticus DSM 3875 - hergestellt nach Beispiel 1a - umgesetzt und es bilden sich nach dünnschichtchromatographischer Analyse - 4 mg D,L-Phenylalaninamid.

### Beispiel 16

Unter den Bedingungen des Beispiels 15 wird das D,L-Aminophenylacetonitril umgesetzt und es bildet sich das D,L-Phenylglycinamid.

### Beispiel 17

Unter den Bedingungen des Beispiels 15 wird das D,L-α-Aminopropionitril umgesetzt und es bildet sich das D,L-Alaninamid.

### Beispiel 18

Unter den Bedingungen des Beispiels 15 wird das D,L-α-Aminovaleriansäurenitril umgesetzt und es bildet sich das D,L-Valinamid.

### Beispiel 19

Unter den Bedingungen des Beispiels 15 wird das D,L-α-Amino-β-methyl-valeriansäurenitril umgesetzt und man erhält das D,L-Isoleucinamid.

### Beispiel 20

Unter den Bedingungen des Beispiels 15 wird das Aminoacetonitril umgesezt und man erhält das Glycinamid.

## Patentansprüche

1. Verfahren zur Herstellung von L-Aminosäuren der allgemeinen Formel I 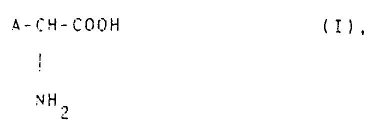 worin
A den Rest eines Aminosäuremoleküls darstellt, aus D,L-α-Aminonitrilen der allgemeinen Formel II 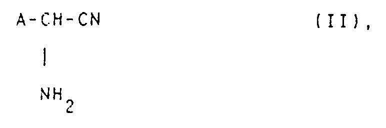 worin
A die oben genannte Bedeutung besitzt, dadurch gekennzeichnet, daß man die α-Amino-nitrile mit einer Kultur von Acinetobacter calcoaceticus DSM 3875 fermentiert und die erhaltenen D,L-Aminosäure-amide der allgemeinen Formel III 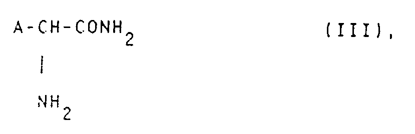 worin
A die oben genannte Bedeutung besitzt mit einer Kultur eines Mikroorganismus der Aminosäure-amid-Racemasen und L-Aminosäureamid-Amidasen besitzt, umsetzt.

2. Verfahren zur Herstellung von Aminosäure-amiden der allgemeinen Formel III 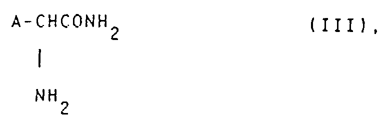 worin
A die oben genannte Bedeutung besitzt, dadurch gekennzeichnet, daß man ein α-Amino-nitril der allgemeinen Formel II 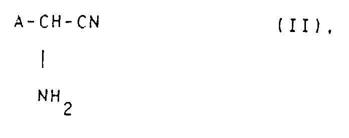 worin
A die oben genannte Bedeutung besitzt, mit einer Kultur von Acinetobacter calcoaceticus DSM 3875 fermentiert.

3. Verfahren zur Herstellung von L-Aminosäuren der allgemeinen Formel I 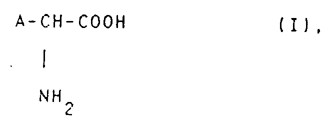 worin
A die oben genannte Bedeutung besitzt, dadurch gekennzeichnet, daß man ein D,L-Aminosäureamid oder ein D-Aminosäure-amid der allgemeinen Formel III 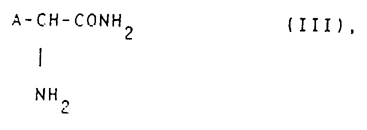 worin
A die oben genannte Bedeutung besitzt, mit einer Kultur eines Mikroorganismus, der Aminosäure-amid-Racemasen und L-Aminosäure-amid-Amidasen besitzt, umsetzt.

## Claims

1. Process for the preparation of L-amino acids of the general formula I 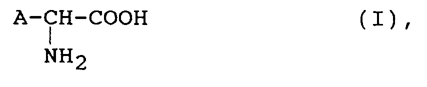 wherein
A represents the residue of an amino acid molecule, from D,L-α-aminonitriles of the general formula II 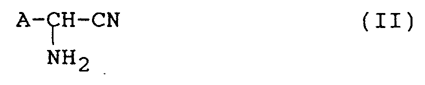 wherein
A is as defined above, characterised in that the α-aminonitriles are fermented with a culture of Acinetobacter calcoaceticus DSM 3875 and the resulting D,L-amino acid amides of the general formula III 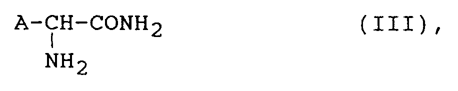 wherein
A is as defined above, are reacted with a culture of a microorganism that possesses amino acid amide racemases and L-amino acid amide amidases.

2. Process for the preparation of amino acid amides of the general formula III 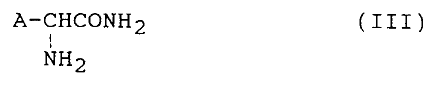 wherein
A is as defined above, characterised in that an α-aminonitrile of the general formula II 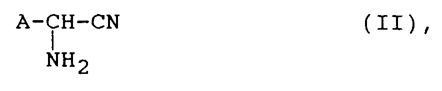 wherein
A is as defined above, is fermented with a culture of Acinetobacter calcoaceticus DSM 3875.

3. Process for the preparation of L-amino acids of the general formula I 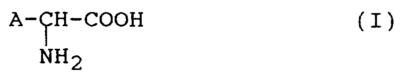 wherein
A is as defined above, characterised in that a D,L-amino acid amide or a D-amino acid amide of the general formula III 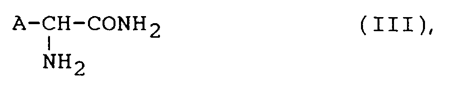 wherein
A is as defined above, is reacted with a culture of a microorganism that possesses amino acid amide racemases and L-amino acid amide amidases.

## Revendications

1. Procédé de préparation de L-aminoacides de formule générale I 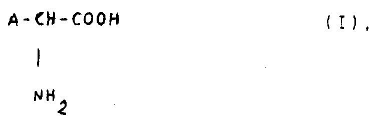 A désignant le radical d'une molécule d'amino-acide, à partir de D,L-α-aminonitriles de formule II 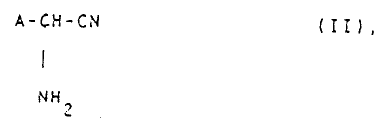 A ayant la signification ci-dessus, procédé caractérisé en ce que l'on fait fermenter les α-aminonitriles avec une culture d'Acinetobacter calcoaceticus DSM 3875, puis on fait réagir les amides de D,L-aminoacides ainsi obtenus, de formule III, 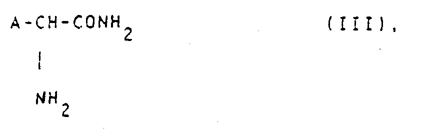 avec une culture d'un micro-organisme possédant à la fois des aminoamides-racémases et des L-aminoamides-amidases.

2. Procédé de préparation d'aminoamides de formule générale III 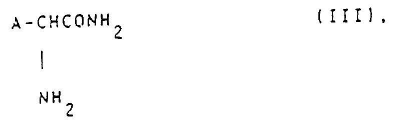 A ayant la même signification qu'à la revendicaiton 1, procédé caractérisé en ce que l'on fait fermenter un α-aminonitrile de formule II 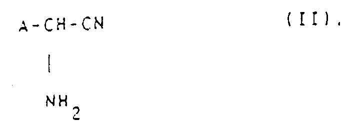 avec une culture d'Acinetobacter calcoaceticus DSM 3875.

3. Procédé de préparation de L-aminoacides de formule générale I telle que définie à la revendication 1 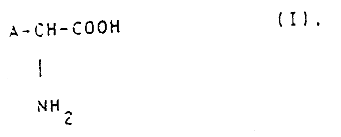 procédé caractérisé en ce que l'on fait réagir un D,L-aminoamide ou un D-aminoamide de formule III 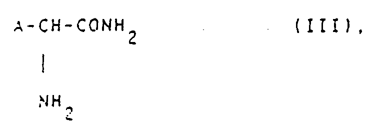 avec une culture d'un micro-organisme possédant des aminoamides-racémases et des L-aminoamides-amidases.
